# FASCICULE DE BREVET EUROPEEN

(11) **EP 3 578 958 B1**
(45) Date de publication et mention de la délivrance du brevet: **05.05.2021**
(21) Numéro de dépôt: 19177300.1
(22) Date de dépôt: 29.05.2019
(51) Int. Cl.: G01N 21/64, G01N 33/04, G01N 33/487, A23C 19/05

(54) **PROCÉDÉ DE DÉTERMINATION DES CASÉINES ET DES PROTÉINES SÉRIQUES DANS DES PRODUITS LAITIERS CRUS OU FAIBLEMENT PASTEURISÉS**
VERFAHREN ZUR BESTIMMUNG DER KASEINE UND DER SERUMPROTEINE IN ROHMILCHPRODUKTEN ODER LEICHT PASTEURISIERTEN MILCHPRODUKTEN
METHOD FOR DETERMINING THE CASEINS AND SERUM PROTEINS IN RAW OR LIGHTLY PASTEURISED DAIRY PRODUCTS

(30) Priorité: 08.06.2018 FR 1855047
(43) Date de publication de la demande: 11.12.2019
(73) Titulaire: Spectralys Innovation, 93230 Romainville (FR)
(72) Inventeur: BIRLOUEZ-ARAGON, Inès, 95120 ERMONT (FR)
(74) Mandataire: Gevers & Orès

(56) Documents cités:
- US-A- 4 271 201
- US-A1- 2017 138 848
- DIEZ ET AL: "Potential of front face fluorescence associated to PLS regression to predict nutritional parameters in heat treated infant formula models", ANALYTICA CHIMICA ACTA, ELSEVIER, AMSTERDAM, NL, vol. 606, no. 2, 17 novembre 2007 (2007-11-17), pages 151-158, XP022387440, ISSN: 0003-2670
- A. ACHARID ET AL: "Potential of front face fluorescence as a monitoring tool of neoformed compounds in industrially processed carrot baby food", LWT- FOOD SCIENCE AND TECHNOLOGY, vol. 49, no. 2, 1 décembre 2012 (2012-12-01), pages 305-311, XP055309481, United Kingdom ISSN: 0023-6438, DOI: 10.1016/j.lwt.2012.06.016
- PIERRE LACOTTE ET AL: "Amaltheys: A fluorescence-based analyzer to assess cheese milk denatured whey proteins", JOURNAL OF DAIRY SCIENCE., vol. 98, no. 10, 1 octobre 2015 (2015-10-01), pages 6668-6677, XP055538493, US ISSN: 0022-0302, DOI: 10.3168/jds.2015-9412
- GLIGUEM H ET AL: "Effects of Sterilization, Packaging, and Storage on Vitamin C Degradation, Protein Denaturation, and Glycation in Fortified Milks", JOURNAL OF DAIRY SCIENCE, AMERICAN DAIRY SCIENCE ASSOCIATION, US, vol. 88, no. 3, 1 mars 2005 (2005-03-01), pages 891-899, XP026964274, ISSN: 0022-0302 [extrait le 2005-03-01]
- RAMÍREZ-PALOMINO P ET AL: "Rapid chromatographic determination of caseins in milk with photometric and fluorimetric detection using a hydrophobic monolithic column", FOOD CHEMISTRY, vol. 142, 1 janvier 2014 (2014-01-01), pages 249-254, XP028706652, ISSN: 0308-8146, DOI: 10.1016/J.FOODCHEM.2013.07.046

## Description

La présente invention se rapporte à un procédé pour évaluer la teneur en protéines d'un aliment. En particulier, l'invention concerne un procédé pour doser simultanément les caséines et les protéines sériques que comprend un aliment protéique liquide tel qu'un lait cru ou faiblement pasteurisé.

### Arrière-plan de l'invention

L'invention peut être appliquée en particulier, mais pas uniquement, aux industries des secteurs laitier et fromager, et notamment pour ce qui concerne la fabrication de fromages, de yaourts, de poudres infantiles, de laits liquides ou encore d'ingrédients laitiers liquides ou en poudre.

Dans le cadre de laits ou de fromages, deux types de protéines sont typiquement présentes: d'une part les caséines, qui sont des micelles insolubles et coagulables sous l'effet de la présure, et d'autre part les protéines sériques, solubles, dans le sérum du lait.

En particulier, l'invention est appliquée aux produits crus ou faiblement traités thermiquement. De tels produits contiennent des protéines sériques qui ne sont pas dénaturées, par exemple sous l'effet de la chaleur, et qui peuvent être précipitées, de même que les protéines coagulables que comprennent ces produits.

Au regard de cette composition, il est connu que la teneur des protéines et des caséines présentes est fondamentale pour déterminer la qualité technologique d'un lait ou d'un fromage comprenant celles-ci, de même que son rendement, sa texture, et plus généralement les qualités du produit fini.

Dans ce contexte, les industriels fromagers et du secteur laitier doivent maîtriser les fluctuations de teneur en caséines du lait, car celles-ci conditionnent directement le rendement fromager. Par exemple, pour garantir une qualité optimale, le fromager doit de préférence respecter un taux constant de protéines et un poids de fromage constant dans chaque produit. Il est donc nécessaire d'anticiper le volume de produit à traiter pour obtenir une quantité de caséines, donc de caillé, équivalente voire égale.

Ainsi, pour maintenir un volume constant de caillé et répondre aux normes réglementaires, les industriels doivent corriger a posteriori le poids de caillé. Il est beaucoup plus rentable d'adapter a priori le volume de lait mis en œuvre en fonction du dosage de caséines, la connaissance de celle-ci assurant la conformité du produit fini.

Par ailleurs, le dosage des protéines sériques permet d'établir le rapport entre ces protéines et les caséines dans un aliment, ce rapport étant défini comme la division mathématique de la teneur en protéines sériques par la teneur en caséines. Or, ce rapport est important à maîtriser pour la fabrication de produits tels que des yaourts ou des formules infantiles, car il influence leur fermeté et texture ou leur digestibilité respectivement. Par ailleurs, le rapport entre protéines sériques et caséines est un critère réglementé pour l'étiquetage des formules infantiles, et doit être renseigné avec précision. Ce rapport conditionne également le rendement fromager si le lait fromager est traité thermiquement, du fait de la précipitation des protéines dénaturées avec les caséines en présence. La connaissance de ce rapport permet ainsi de cibler la température de pasteurisation du produit, pour maîtriser la proportion de protéines sériques qui sera dénaturée, et donc les valeurs souhaitées du rapport protéines sériques dénaturées sur caséines.

Aujourd'hui, une méthode connue et normée, la méthode Kjeldahl, pour doser les caséines et les protéines sériques comprend l'utilisation d'appareils et de solvants, ce qui oblige à une pratique en laboratoire. Le résultat de l'analyse est donc différé par rapport à la prise de l'échantillon issu de l'aliment, et ne permet donc pas d'adapter les processus de traitement ce celui-ci en temps utile.

Une autre méthode connue vise à doser les protéines totales par une analyse spectroscopique dans l'infrarouge et/ou dans le proche infrarouge. Mettant en œuvre une calibration complexe à adapter de manière fréquente, cette méthode permet de doser les caséines d'un lait. Toutefois, cette méthode ne permet pas de doser les protéines sériques dénaturables, et qui sont d'intérêt nutritionnel. En réalisant la soustraction mathématique entre la teneur en protéines totales et la teneur en caséines, ces deux teneurs étant mesurées par spectroscopie infrarouge et/ou proche infrarouge, on obtient la somme des teneurs de protéines sériques solubles et de fractions protéose peptone, non dénaturable, et sans interaction connue avec les caséines.

En particulier, il est connu que l'utilisation de la spectroscopie de fluorescence permet de mesurer les protéines sériques d'un échantillon, par exemple un échantillon de lait cru, grâce à une précipitation des caséines à l'aide d'un tampon adéquat. Au moyen d'un spectromètre, il est dès lors possible de mesurer la fluorescence d'acides aminés marqueurs de la concentration protéique, et en particulier la fluorescence de tryptophane, composant des protéines sériques dénaturables, au sein de l'échantillon.

Par ailleurs, il est connu des méthodes visant à mesurer la teneur en protéines totales dans des échantillons par fluorescence après avoir réalisé une transparisation de ceux-ci. Ces méthodes permettent de calculer le taux de caséines par soustraction entre le taux de protéines totales et le taux de protéines solubles.

Les demandes de brevet FR 2 752 941 et FR et FR 3 019 299 décrivent des exemples de méthodes reposant sur l'utilisation d'une mesure de fluorescence à angle droit en milieu transparent pour déterminer la teneur en protéines et/ou en caséines d'un échantillon. L'une de ces méthodes consiste à précipiter un échantillon de lait au moyen d'un tampon acide. Après environ 5 minutes, une mesure de fluorescence à angle droit permet d'en déduire la teneur en protéines totales avec une erreur de l'ordre 3%. Par ailleurs, une autre méthode serait de transpariser un échantillon de lait au moyen d'un tampon de force ionique, d'impact déstabilisant des micelles de caséines et de pH approprié. Après environ 3 minutes, une mesure de fluorescence à angle droit est ensuite réalisée pour en déduire la teneur en protéines totales avec une erreur de l'ordre 5%. Sur la base d'un même échantillon de lait, la soustraction des teneurs ainsi mesurées de protéines totales et de protéines solubles permet d'en déduire la teneur en caséines.

Cependant, la méthode Kjeldahl est complexe, polluante et chère et cette dernière méthode reste relativement lourde à mettre en œuvre dans un contexte industriel. Notamment, cette dernière méthode de l'art antérieur présente trois inconvénients majeurs. Premièrement, elle est sont lente puisqu'environ 8 minutes sont nécessaires à la mesure du taux de caséines. Deuxièmement, la soustraction de la teneur en protéines totales et de la teneur en protéines solubles présente une imprécision importante de l'ordre de 8%, par addition cumulée des erreurs. Troisièmement, ces méthodes sont relativement peu ergonomiques, puisque deux préparations d'échantillons sont nécessaires.

### Objet et résumé de l'invention

Afin de pallier l'un au moins des inconvénients précités, un objet de la présente invention est de proposer une méthode pour doser simultanément les caséines et les protéines sériques que comprend un aliment protéique liquide tel qu'un lait cru ou faiblement pasteurisé.

Dans ce but, un objet de l'invention concerne un procédé de détermination d'une teneur en caséines et d'une teneur en protéines sériques dans un aliment liquide tel qu'un lait cru ou faiblement pasteurisé, qui comprend les étapes successives consistant à :
a) former une solution dudit aliment liquide en ajoutant un tampon de pH prédéterminé, ladite solution comprenant lesdites protéines sériques et lesdites caséines, les caséines étant sous forme coagulée en suspension dans la solution et les protéines sériques sont solubilisées dans la solution ;
b) agiter la solution pour former un mélange homogène des protéines sériques et des caséines ;
c) acquérir une pluralité de spectres de fluorescence frontale dudit mélange, lesdits spectres de fluorescence frontale résultant d'une excitation dudit mélange homogène par au moins un rayonnement électromagnétique de longueur d'onde déterminée ;
d) diluer le mélange en ajoutant une quantité prédéterminée d'eau de façon à rendre le mélange transparent et à séparer les protéines sériques des caséines;
e) acquérir une pluralité de spectres de fluorescence à angle droit dudit mélange dilué, lesdits spectres de fluorescence à angle droit résultant d'une excitation du mélange dilué par au moins un rayonnement électromagnétique de longueur d'onde déterminée ; et
f) déterminer la teneur en caséines et la teneur en protéines sériques de l'aliment liquide par application d'une analyse multivoies aux spectres de fluorescence frontale et aux spectres de fluorescence à angle droit.

Selon différentes caractéristiques supplémentaires qui pourront être prises ensemble ou de façon séparée :
- une longueur d'onde d'au moins un rayonnement électromagnétique est comprise entre 270 nanomètres et 340 nanomètres, et de préférence égale à 280 nanomètres ;
- ledit tampon est un tampon d'acétate de sodium et/ou présente un pH compris entre 4,50 et 4,65, de préférence égal à 4,55 ou à 4,6 ;
- l'acquisition d'au moins un spectre de fluorescence frontale au cours de l'étape c) et/ou d'au moins un spectre de fluorescence à angle droit au cours de l'étape e) est effectuée par une mesure de la diffusion par fluorescence de tryptophane présent dans le mélange et/ou dans le mélange dilué ;
- l'aliment liquide et le tampon ajouté lors de l'étape a) présentent des volumes tels que le rapport entre le volume de l'aliment liquide et le volume du tampon est compris entre 1 et 5, et est de préférence égal à 3 ;
- l'étape b) d'agitation est mise en œuvre au moyen d'un vortex ; et
- ladite analyse multivoies est une analyse PARAFAC appliquée à une méthode de régression choisie parmi une méthode de régression multilinéaire ou une méthode de régression par moindres carrés partiels.

### Brève description des dessins

D'autres caractéristiques et avantages de la présente invention ressortiront de la description faite ci-dessous, en référence aux dessins annexés qui en illustrent des exemples de réalisation dépourvus de tout caractère limitatif.

Sur les figures :

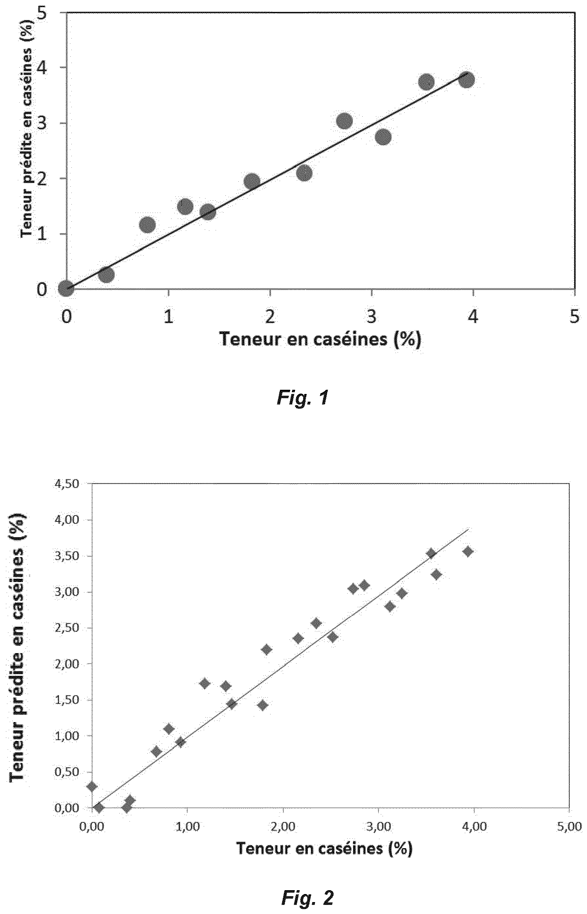

- la **figure 1** est un graphique illustrant des valeurs en pourcentage de teneurs de caséines de plusieurs échantillons de lait prédites par un modèle de régression multilinéaire (en ordonnées) lorsque les étapes d'un procédé selon l'invention sont mises en œuvre, représentées en fonction de valeurs de teneurs de caséines de référence (en abscisses) ; et
- la **figure 2** est un graphique illustrant des valeurs en pourcentage de teneurs de caséines de plusieurs autres échantillons de lait prédites par un modèle de régression multilinéaire (en ordonnées) lorsque les étapes d'un procédé selon l'invention sont mises en oeuvre, représentées en fonction de valeurs de teneurs de caséines de référence (en abscisses).

Naturellement, pour satisfaire des besoins spécifiques, une personne compétente dans le domaine de la technique pourra appliquer des modifications dans la description suivante. Bien qu'elle se réfère à différents modes de réalisation, la présente invention n'est pas limitée à ces modes de réalisation spécifiques, et toutes modifications propres au champ d'application de la présente invention dans le cadre des revendications jointes peuvent être considérées comme évidentes pour une personne versée dans l'art de la technique correspondant.

### Description détaillée d'un mode de réalisation

Une première étape du procédé selon l'invention consiste à prélever un volume d'un aliment liquide auquel on ajoute un tampon concentré.

Dans un exemple de mise en œuvre de l'invention, on prépare et traite un échantillon de mesure d'un lait cru comme suit. Un volume de 7,5 millilitres de lait est mélangé à un volume de 2,5 millilitres d'un tampon de pH égal à 4,55. De manière non limitative, le volume de lait mélangé au tampon est compris entre 1 millilitre et 10 millilitres, mais peut également être choisi de sorte à ce que le pH du mélange du volume de lait et du volume de tampon reste aussi proche que possible de celui du tampon initial, 4,55 dans le cas présent.

De préférence, le tampon est un tampon d'acétate de sodium. Selon d'autres modes de réalisation de l'invention, le tampon est un tampon de phosphate de sodium ou de potassium. Selon encore d'autres modes de réalisation de l'invention, le pH du tampon est compris entre 4,5 et 4,65.

De préférence, le volume de lait et le volume de tampon sont sélectionnés de sorte à ne pas diluer l'échantillon de manière excessive. En particulier, on privilégie un mélange dans lequel le rapport du volume du tampon et du volume du lait est compris entre deux dixièmes et trois dixièmes.

Sous l'effet de ce tampon, les caséines coagulent pour former un précipité dense de protéines coagulables de l'aliment, ce précipité se trouvant en suspension dans une solution comprenant les protéines sériques solubles du lait initial.

L'ensemble est ensuite agité, par exemple au moyen d'un vortex, pour obtenir un mélange dans lequel le précipité et la solution sont homogènes. Une agitation par vortex est avantageuse vis-à-vis d'une agitation par centrifugation par exemple, puisqu'elle évite aux composants du mélange de s'accoler dans le récipient contenant celui-ci, auquel cas il serait plus difficile de récupérer une partie de ceux-ci. Ce mélange est ensuite versé dans une cuvette de capacité suffisante pour accueillir celui-ci.

Après coagulation des caséines, les caséines contenues dans le lait ont tendance à diffuser la lumière tandis que les protéines sériques sont transparentes et non-diffusantes. Ainsi, on peut établir que les caséines sont responsables de 99% de la lumière émise, tandis que les protéines sériques n'interfèrent quasiment pas du tout avec celle-ci puisqu'elles sont transparentes et non diffusantes.

Pour ce faire, on réalise une acquisition d'un ou de plusieurs spectres de fluorescence frontale émis par le mélange, par exemple au moyen de l'illumination de l'échantillon par une source de lumière monochromatique dont la longueur d'onde est comprise entre 270 et 300 nanomètres, et de préférence égale à 280 nanomètres. Les rayonnements de fluorescence peuvent être récoltés par un spectromètre, par exemple un spectromètre comprenant une caméra CCD. Avantageusement, une mesure de la fluorescence frontale permet de déterminer l'entièreté de l'empreinte du mélange.

Les longueurs d'onde des spectres électromagnétiques acquis au cours des étapes du procédé de l'invention sont de préférence comprises entre 270 nanomètres et 300 nanomètres.

Dans la présente invention, le tryptophane est utilisé comme marqueur de la teneur en protéines sériques solubles dans un aliment analysé. De préférence, le tryptophane présent dans le surnageant est dosé par fluorescence. La fluorescence du tryptophane permet ainsi de déterminer la concentration en protéines solubles faiblement dénaturées et est donc fortement corrélée à la teneur en protéines.

Avantageusement, le dosage du tryptophane émis par les caséines présentes est optimal pour une longueur d'onde de 280 nanomètres. Le dosage du tryptophane pourrait être également effectué par son absorption dans l'ultraviolet.

Selon d'autres modes de réalisation, une mesure de fluorescence peut également être envisagée au moyen d'au moins une longueur d'onde supérieure à 300 nanomètres, par exemple 360 nanomètres, en vue de récolter des informations supplémentaires.

Selon un mode de réalisation de l'invention, une ou plusieurs autres mesures peuvent être effectuées. Dans ce cas, on agite à nouveau le mélange pour garantir l'homogénéité du précipité et de la solution.

A la fin de l'application du procédé selon l'invention, la mesure de la fluorescence frontale permet d'estimer rapidement, en environ une minute et avec une erreur inférieure à 3%, la teneur en caséines de l'aliment.

Une étape suivante du procédé selon l'invention consiste à rendre transparent un échantillon du mélange en le diluant avec une quantité prédéterminée d'eau. Avantageusement, cet ajout d'eau permet de séparer les protéines sériques des caséines précipitées. Par ailleurs, la transparisation de l'échantillon du mélange par une quantité prédéterminée d'eau permet également, selon une étape correspondante, de le filtrer. On évite ainsi une perte des protéines ou des caséines qui resteraient collées dans un dispositif, par exemple une cuvette qui contiendrait l'échantillon et le mélange.

Une étape suivante du procédé selon l'invention consiste à acquérir une pluralité de spectres de fluorescence à angle droit, également appelée fluorescence conventionnelle, du mélange dilué. Les spectres de fluorescence à angle droit résultent de l'excitation du mélange dilué par au moins un rayonnement électromagnétique de longueur d'onde sélectionnée.

Selon un autre mode de réalisation de l'invention, la mesure de la fluorescence frontale est mesurée pour une première longueur d'onde d'excitation de 280 nanomètres, une deuxième longueur d'onde d'excitation de 340 nanomètres. Avantageusement, l'utilisation de plusieurs longueurs d'onde d'excitation permet de récupérer une pluralité correspondante de spectres d'émission, ce qui permet de fournir des informations supplémentaires quant aux teneurs en caséines, en protéines sériques voire en d'autres composants de l'aliment. Par ailleurs, la réalisation d'une mesure pour une longueur d'onde d'émission comprise entre 330 et 350 nanomètres permet avantageusement d'obtenir un signal de sensibilité maximale.

On rappelle que la diffusion (dénommée diffusion de Rayleigh, lorsque la dimension des particules de la matrice est proche de la longueur d'onde d'excitation, ou diffusion de Mie, lorsque celle-ci est très au-dessus de la longueur d'onde), correspond à une interaction élastique (sans perte d'énergie) entre la lumière à la longueur d'onde considéré et la matrice. Cette interaction rend compte de la structure de la matrice et notamment de la taille et de la concentration des particules de dimension proche de la longueur d'onde.

Selon un mode de réalisation du procédé l'invention, on prévoit une étape de filtrage de la diffusion, qui permet avantageusement d'éviter de polluer la mesure de fluorescence à angle droit.

La figure 1 représente une première application de l'invention à plusieurs échantillons de lait.

Après avoir préparé un volume de chaque échantillon pour obtenir un mélange homogène d'un précipité de caséines en suspension dans une solution qui contient des protéines solubles, on a mesuré la fluorescence en mode frontal de ce mélange au moyen de deux longueurs d'onde d'excitation, l'une étant égale à 280 nanomètres et l'autre étant égale à 340 nanomètres.

Les données spectrales recueillies pour chaque échantillon ont ensuite été organisées en une matrice de grandes dimensions dite « matrice excitation-émission » (MEE), dont une dimension représente les longueurs d'onde d'émission et l'autre dimension représente les longueurs d'onde d'émission. La MEE ainsi obtenue a ensuite été analysée par une méthode d'analyse mathématique, ce qui permet d'extraire des informations correspondant aux profils bilinéaires de fluorescence et à leurs intensités relatives. Ensuite, une méthode de régression linéaire multiple a été appliquée pour prédire la valeur de la teneur en caséines pour chaque échantillon de l'aliment.

En vue de mettre en œuvre l'invention, une phase de calibration peut être réalisée préalablement. Avant de déterminer la teneur en caséines d'un échantillon donné au moyen du procédé de l'invention, cette calibration peut être mise en oeuvre au moyen une analyse mathématique. Comme premier exemple, une analyse par composantes principales (PCA) ou encore une analyse de régression par les moindres carrés partiels (PLS) est mise en œuvre lorsqu'une seule longueur d'onde d'excitation est utilisée. Comme deuxième exemple, une analyse de type PARAFAC ou tout autre méthode de décomposition multivoie est mise en œuvre si plus qu'une longueur d'onde d'excitation est utilisée.

En référence à la figure 1, chaque échantillon comporte une teneur en caséines augmentant progressivement. Dans cette première application, on a ajouté à un échantillon de lait différents volumes d'une poudre de caséines hautement concentrée tout en maintenant constante la teneur en protéines solubles.

Sur la figure 1, une modélisation simple par régression multilinéaire ou encore de régression par les moindres carrés partiels (PLS) est applicable après décomposition des matrices excitation-émission pour déterminer une teneur en caséines. Une modélisation de type PARAFAC est applicable si l'échantillon est éclairé par plusieurs rayonnements lumineux ou par une source de lumière continue. La valeur prédite ainsi obtenue est représentée en ordonnées en fonction des valeurs de référence de caséines, exprimée en gramme de caséine pour 100 grammes d'échantillon, afin de construire la calibration. Les résultats obtenus montre que la calibration Comme indiqué, la calibration montre que l'on a obtenu une racine carrée de l'erreur d'étalonnage égale à 0,283%, et une racine-carréee de l'erreur de validation croisée égale à 0,320%, ce qui correspond à une erreur relative égale à 10%.

Ces résultats illustrent l'excellente corrélation existant entre les teneurs en caséines obtenues au moyen du procédé selon l'invention appliqué à divers échantillons de lait, et les teneurs de référence.

On a également testé une deuxième application de l'invention à plusieurs échantillons de lait, chacun de ces échantillons comportant une teneur en protéines solubles augmentant progressivement. Dans cette deuxième application, on a ajouté à un échantillon de lait différents volumes d'une poudre de protéines sériques solubles tout en maintenant constante la teneur en protéines solubles.

Pour tous les échantillons de cette application, la concentration moyenne en caséines a été quantifiée comme étant de 0,25% avec une déviation standard de 3%, qui est compatible avec l'erreur de reproductibilité des mesures. La fraction de protéines solubles a été mesurée et a confirmé des résultats fiables et cohérents avec ceux de la demande de brevet FR 2 752 941.

La figure 2 représente une troisième application de l'invention à plusieurs échantillons, chaque échantillon comportant un volume d'un rétentat de lait microfiltré augmentant progressivement, de sorte à avoir différents échantillons dont les teneurs en caséines et en protéines sériques solubles est fortement variable.

Après homogénéisation de chaque échantillon, les mesures de fluorescence en mode frontal correspondantes ont été effectuées par illumination de chacun de ceux-ci au moyen d'une première longueur d'onde de 280 nanomètres et d'une deuxième longueur d'onde de 340 nanomètres.

En utilisant une méthode de décomposition PARAFAC suivie d'une méthode de régression multilinéaire, les résultats représentés sur la figure 2 montrent qu'on obtient une calibration fiable de la valeur prédite de la teneur en caséines de chaque échantillon (en ordonnées) par rapport aux valeurs de référence de la teneur caséines exprimée en gramme de caséine pour 100 grammes d'échantillon.

Sur la base d'une régression linéaire dont l'équation de la courbe est définie par y(x) - 0,984 x, les résultats de la calibration montrent qu'on obtient une racine carrée de l'erreur d'étalonnage égale à 0,34%, ce qui met en évidence la plus grande variabilité des échantillons dans cette troisième application. Par ailleurs, l'obtention d'une racine-carrée de l'erreur de validation croisée égale à 0,56% indique qu'une calibration robuste nécessite, idéalement, un plus grand nombre d'échantillons. Néanmoins, du fait de la rapidité de ce procédé, plusieurs centaines d'échantillons peuvent être mesurés par jour.

## Revendications

1. Procédé de détermination d'une teneur en caséines et d'une teneur en protéines sériques dans un aliment liquide tel qu'un lait cru ou faiblement pasteurisé, qui comprend les étapes successives consistant à :
a) former une solution dudit aliment liquide en ajoutant un tampon de pH prédéterminé, ladite solution comprenant lesdites protéines sériques et lesdites caséines, les caséines étant sous forme coagulée en suspension dans la solution et les protéines sériques sont solubilisées dans la solution ;
b) agiter la solution pour former un mélange homogène des protéines sériques et des caséines ;
c) acquérir une pluralité de spectres de fluorescence frontale dudit mélange, lesdits spectres de fluorescence frontale résultant d'une excitation dudit mélange homogène par au moins un rayonnement électromagnétique de longueur d'onde déterminée ;
d) diluer le mélange en ajoutant une quantité prédéterminée d'eau de façon à rendre le mélange transparent et à séparer les protéines sériques des caséines;
e) acquérir une pluralité de spectres de fluorescence à angle droit dudit mélange dilué, lesdits spectres de fluorescence à angle droit résultant d'une excitation du mélange dilué par au moins un rayonnement électromagnétique de longueur d'onde déterminée ; et
f) déterminer la teneur en caséines et la teneur en protéines sériques de l'aliment liquide par application d'une analyse multivoies aux spectres de fluorescence frontale et aux spectres de fluorescence à angle droit.

2. Procédé selon la revendication 1, dans lequel une longueur d'onde d'au moins un rayonnement électromagnétique est comprise entre 270 nanomètres et 340 nanomètres, et de préférence égale à 280 nanomètres.

3. Procédé selon la revendication 1 ou 2, dans lequel ledit tampon est un tampon d'acétate de sodium et/ou présente un pH compris entre 4,50 et 4,65, de préférence égal à 4,55 ou à 4,6.

4. Procédé selon l'une quelconque des revendications précédentes, dans lequel l'acquisition d'au moins un spectre de fluorescence frontale au cours de l'étape c) et/ou d'au moins un spectre de fluorescence à angle droit au cours de l'étape e) est effectuée par une mesure de tryptophane présent dans le mélange et/ou dans le mélange dilué.

5. Procédé selon l'une quelconque des revendications précédentes, dans lequel l'aliment liquide et le tampon ajouté lors de l'étape a) présentent des volumes tels que le rapport entre le volume de l'aliment liquide et le volume du tampon est compris entre 1 et 5, et est de préférence égal à 3.

6. Procédé selon l'une quelconque des revendications précédentes, dans lequel l'étape b) d'agitation est mise en œuvre au moyen d'un vortex.

7. Procédé selon l'une quelconque des revendications précédentes, dans lequel ladite analyse multivoies est une analyse PARAFAC appliquée à une méthode de régression choisie parmi une méthode de régression multilinéaire ou une méthode de régression par moindres carrés partiels.

## Patentansprüche

1. Verfahren zur Bestimmung eines Kaseingehalts und eines Serumproteingehalts in einem flüssigen Lebensmittel, wie einer Rohmilch oder leicht pasteurisierten Milch, das die aufeinanderfolgenden Schritte umfasst, die bestehen aus:
a) Bilden einer Lösung aus dem flüssigen Lebensmittel durch Hinzufügen eines Puffers mit vorbestimmtem pH-Wert, wobei die Lösung die Serumproteine und die Kaseine umfasst, wobei die Kaseine in geronnener Form in Suspension in der Lösung vorliegen und die Serumproteine in der Lösung löslich gemacht sind;
b) Schütteln der Lösung, um ein homogenes Gemisch aus den Serumproteinen und den Kaseinen zu bilden;
c) Erfassen einer Vielzahl von Spektren frontaler Fluoreszenz aus dem Gemisch, wobei sich die Spektren frontaler Fluoreszenz aus einer Anregung des homogenen Gemischs durch mindestens eine elektromagnetische Strahlung mit bestimmter Wellenlänge ergeben;
d) Verdünnen des Gemischs durch Hinzufügen einer vorbestimmten Menge an Wasser, um das Gemisch durchsichtig zu machen, und die Serumproteine und Kaseine zu trennen;
e) Erfassen einer Vielzahl von Spektren rechtwinkliger Fluoreszenz aus dem verdünnten Gemisch, wobei sich die Spektren rechtwinkliger Fluoreszenz aus einer Anregung des verdünnten Gemischs durch mindestens eine elektromagnetische Strahlung mit bestimmter Wellenlänge ergeben; und
f) Bestimmen des Kaseingehalts und des Serumproteingehalts des flüssigen Lebensmittels durch Anwendung einer Mehrweganalyse an den Spektren frontaler Fluoreszenz und an den Spektren rechtwinkliger Fluoreszenz.

2. Verfahren nach Anspruch 1, wobei eine Wellenlänge mindestens einer elektromagnetischen Strahlung zwischen 270 Nanometer und 340 Nanometer enthalten ist und vorzugsweise gleich 280 Nanometer ist.

3. Verfahren nach Anspruch 1 oder 2, wobei der Puffer ein Natriumacetat-Puffer ist und/oder einen pH-Wert aufweist, der zwischen 4,50 und 4,65 enthalten ist, vorzugsweise gleich 4,55 oder 4,6 ist.

4. Verfahren nach einem der vorstehenden Ansprüche, wobei die Erfassung mindestens eines Spektrums frontaler Fluoreszenz im Verlauf von Schritt c) und/oder mindestens eines Spektrums rechtwinkliger Fluoreszenz im Verlauf von Schritt e) durch eine Messung von im Gemisch und/oder im verdünnten Gemisch vorhandenem Tryptophan erfolgt.

5. Verfahren nach einem der vorstehenden Ansprüche, wobei das flüssige Lebensmittel und der im Schritt a) hinzugefügte Puffer derartige Volumina aufweisen, dass das Verhältnis zwischen dem Volumen des flüssigen Lebensmittels und dem Volumen des Puffers zwischen 1 und 5 enthalten ist, und vorzugsweise gleich 3 ist.

6. Verfahren nach einem der vorstehenden Ansprüche, wobei der Schritt b) des Schüttelns mittels einer Verwirbelung umgesetzt wird.

7. Verfahren nach einem der vorstehenden Ansprüche, wobei die Mehrweganalyse eine PARAFAC-Analyse ist, die an ein Regressionsverfahren angewendet wird, das ausgewählt ist aus einem multilinearen Regressionsverfahren oder einem Regressionsverfahren der partiellen kleinsten Quadrate.

## Claims

1. Method for determining a casein content and a serum protein content in a liquid food such as raw or lightly pasteurised milk, which comprises the successive steps consisting of:
a) forming a solution of said liquid food by adding a buffer of predetermined pH, said solution comprising said serum proteins and said caseins, the caseins being in coagulated form in suspension in the solution and the serum proteins are solubilised in the solution;
b) stirring the solution in order to form a homogeneous mixture of the serum proteins and caseins;
c) acquiring a plurality of front fluorescence spectra of said mixture, said front fluorescence spectra resulting from an excitation of said homogeneous mixture by at least one electromagnetic radiation of given wavelength;
d) diluting the mixture by adding a predetermined quantity of water so as to make the mixture transparent and to separate the serum proteins from the caseins;
e) acquiring a plurality of right-angle fluorescence spectra of said diluted mixture, said right-angle fluorescence spectra resulting from an excitation of the diluted mixture by at least one electromagnetic radiation of given wavelength; and
f) determining the casein content and the serum protein content of the liquid food by applying a multichannel analysis to the front fluorescence spectra and to the right-angle fluorescence spectra.

2. Method according to claim 1, wherein a wavelength of at least one electromagnetic radiation is between 270 nanometres and 340 nanometres, and preferably equal to 280 nanometres.

3. Method according to claim 1 or 2, wherein said buffer is a sodium acetate buffer and/or has a pH of between 4.50 and 4.65, preferably equal to 4.55 or 4.6.

4. Method according to any one of the preceding claims, wherein the acquisition of at least one front fluorescence spectrum during step c) and/or of at least one right-angle fluorescence spectrum during step e) is carried out by measuring tryptophan present in the mixture and/or in the diluted mixture.

5. Method according to any one of the preceding claims, wherein the liquid food and the buffer added during step a) have volumes such that the ratio between the volume of the liquid food and the volume of the buffer is between 1 and 5, and is preferably equal to 3.

6. Method according to any one of the preceding claims, wherein the stirring step b) is implemented by means of a vortex.

7. Method according to any one of the preceding claims, wherein said multichannel analysis is a PARAFAC analysis applied to a regression method selected from a multilinear regression method or a partial least squares regression method.
